# EUROPEAN PATENT SPECIFICATION

(11) **EP 4 205 203 B1**
(45) Date of publication and mention of the grant of the patent: **06.08.2025**
(21) Application number: 21790258.4
(22) Date of filing: 23.08.2021
(51) Int. Cl.: H01M 8/0239, C07D 285/12

(54) **2,5-DIMERCAPTO-1,3,4-THIADIAZOLE ("DMTD") METAL SALT DERIVATIVES**
2,5-DIMERCAPTO-1,3,4-THIADIAZOL-METALLSALZDERIVATE
DÉRIVÉS DE SELS MÉTALLIQUES DE 2,5-DIMERCAPTO-1,3,4-THIADIAZOLE ("DMTD")

(30) Priority: 26.08.2020 US 202063070562 P
(43) Date of publication of application: 05.07.2023
(73) Proprietor: The Lubrizol Corporation, Wickliffe, Ohio 44092-2298 (US); Ohio State Innovation Foundation, Columbus, OH 43201 (US)
(72) Inventor: ADAMS, Paul E., Wickliffe, Ohio 44092-2298 (US); RASIK, Christopher M., Wickliffe, Ohio 44092-2298 (US); ZHANG, Shiyu, Columbus, Ohio 43210 (US); DAVIS, Shelby, Columbus, Ohio 43210 (US); TUTTLE, Madison, Columbus, Ohio 43210 (US); KONRAD, Ryan Richard, Wickliffe, Ohio 44092-2298 (US)
(74) Representative: D Young & Co LLP
(86) International application number: PCT/US2021/047135
(87) International publication number: WO 2022/046625

(56) References cited:
- WO-A1-2021/055275
- WO-A2-2020/108787
- US-A- 4 762 848
- US-A1- 2015 267 113
- US-A1- 2016 168 724
- US-A1- 2018 072 669

## Description

### BACKGROUND OF THE INVENTION

The disclosed technology relates to a redox flow battery electrolyte comprising a polar solvent, and a metal salt of 2,5-dimercapto-1,3,4-thiadiazole.

Some simple alkyl and aryl substituted DMTD derivatives are known, for example, as taught in US 2,736,729, granted Feb. 28, 1956 to Krzikalla et al. and US3,212,892, granted Oct. 19, 1965, to von König et al. Simple acrylic acid alkyl and aryl esters of DMTD are also known, for example, as taught in JP 2013234126, published Nov. 21, 2013 to Oya et al., and US 5,258,395, granted Nov. 2, 1993 to Murase et al. US 2018/072669 A1 describes aqueous organic redox flow batteries that include a first redox active material that can include a metallocene or a salt thereof, and a second redox active material that can include a viologen or a salt thereof. The aqueous organic redox flow batteries may further include a first aqueous electrolyte, a second aqueous electrolyte, and a separator between the first and second aqueous electrolytes. Also disclosed are methods of making the metallocene and viologen compounds. WO 2020/108787 A2 relates to an aqueous electrolyte solution with a temperature of at least 30°C containing a compound having at least one redox-active moiety of formula I described therein. The electrolyte can be used in redox flow batteries.

New chemistries are needed that can readily change oxidation states and are based on organic chemistry. Examples of such chemistry in the art usually start with some or all of the relatively expensive starting materials and reagents, may need multiple steps to obtain the target final product, generate large quantities of undesirable process wastes, and could also require tedious purification steps.

### SUMMARY OF THE INVENTION

The disclosed technology, therefore, solves the problem of difficult to produce and expensive but readily oxidizable organic chemistry by providing new DMTD metal salts that can be synthesized in only one or two steps, starting from readily available and inexpensive raw materials, use processing that generates little or no waste, and involve reactions which proceed rapidly and in high product conversions.

The present invention is defined in the appended claim set. In particular, the technology provides a redox flow battery electrolyte comprising A) a polar solvent, and B) a DMTD derivative of formula (I): 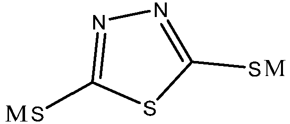 where M is H or an alkali or alkaline earth metal, such as Li, Na, K, Mg, or Ca, with the proviso that at least one of M is an alkaline or alkaline earth metal. In some embodiments, both M in the foregoing formula can be an alkaline earth metal.

In particular embodiments, the polar solvent in the electrolyte can be water.

In some embodiments, the electrolyte can include a supporting redox active species. In particular, the electrolyte can include at least one water soluble mono-alkylated DMTD derivative. The supporting redox active species can also be at least one of a lithium salt, potassium salt, sodium salt, ammonium salt, and combinations thereof.

The technology also includes a redox flow battery system comprising A) a catholyte comprising the redox flow battery electrolyte discussed herein containing the DMTD metal salt derivatives and optional mono-alkylated DMTD derivatives, and B) an anolyte comprising a redox active species other than the DMTD metal salt derivative, or a mixture thereof in a polar solvent.

### BRIEF DESCRIPTION OF THE FIGURES

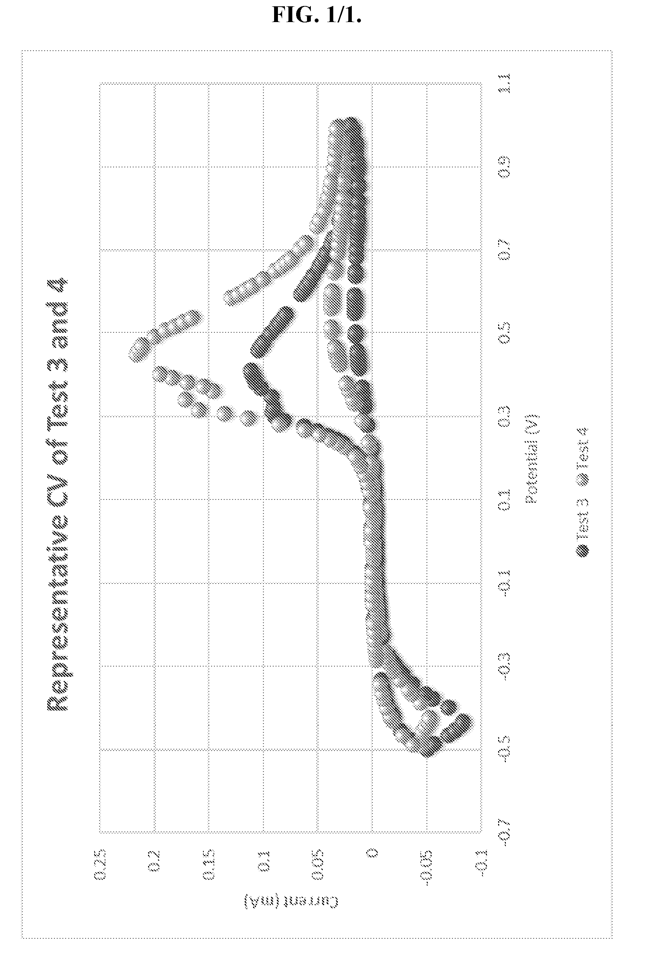

FIG 1 is the CV plots for Tests 3 and 4 from Table 1.

### DETAILED DESCRIPTION OF THE INVENTION

Various preferred features and embodiments will be described below by way of non-limiting illustration.

Provided herein are metal salt derivatives of 2,5-Dimercapto-1,3,4-thiadiazole ("DMTD") represented in the pure form by formula I: 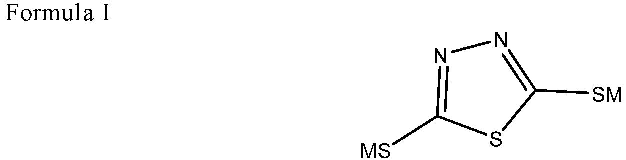 where M is H or an alkali or alkaline earth metal, with the proviso that at least one of M is an alkali or alkaline earth metal.

At least one M is an alkali or alkaline earth metal. The alkali or alkaline earth metal can be any of the alkali or alkaline earth metals. Alkali metals can include lithium, sodium, potassium, rubidium, cesium, and francium. Alkaline earth metals can include beryllium, magnesium, calcium, strontium, barium and radium. In an embodiment at least one M in Formula I can be lithium, sodium, potassium, magnesium, and calcium. At least one M in Formula I can be lithium. At least one M in Formula I can be sodium. At least one M in Formula I can be potassium. At least one M in Formula I can be magnesium. At least one M in Formula I can be calcium. Both M in Formula I can be an alkali or alkaline earth metal, and can be the same or different alkali or alkaline earth metals.

The DMTD metal salt derivatives may be prepared with an alkali or alkaline earth metal hydroxide or alkoxide. Examples of an alkali or alkaline earth metal hydroxide include lithium hydroxide, sodium hydroxide, potassium hydroxide, magnesium hydroxide, and calcium hydroxide. Examples of alkali or alkaline earth metal alkoxides include sodium methoxide, potassium t-butoxide, potassium ethoxide, and lithium methoxide. To obtain a derivative with both M as an alkali or alkaline earth metal, an excess of alkali or alkaline earth metal hydroxide or alkoxide can be employed, such as, for example, 2 molar equivalents or more of alkali or alkaline earth metal hydroxide or alkoxide per molar equivalent DMTD. To obtain a derivative with just one M as an alkali or alkaline earth metal, an equivalent amount of alkali or alkaline earth metal hydroxide or alkoxide can be employed, that is, 1 molar equivalent of alkali or alkaline earth metal hydroxide or alkoxide per molar equivalent DMTD.

The present technology also includes an electrolyte useful in redox flow batteries ("RFB"). The electrolyte includes A) a polar solvent, and B) the aforementioned 2,5-Dimercapto-1,3,4-thiadiazole ("DMTD") metal salt derivatives.

The polar solvent can be any solvent in which the DMTD or derivative thereof is soluble in both its oxidized and reduced states. The solvent should also remain inert within the working conditions of the RFB. It is expected that those of ordinary skill can readily determine the solubility and inertness of, and therefore choose, the appropriate solvent. The simplest solvent for the present technology could be water.

The polar solvent can also be, for example, an alcohol, such as C₁ to C₁₀ alcohol or glycol, including, for example, methanol, ethanol, 1-propanol, 2-propanol, ethylene glycol, propylene glycol. Ethers may also be employed as the solvent, including, for example, dimethoxymethane, methoxybenzene (anisole), tetrahydrofuran (THF), 2-methyltetrahydrofuran, 1,4-dioxane, 1,3-dioxolane (DOL), 4-methyl-1,3-dioxolane, 1,2-dimethoxyethane (DME), and bis(2-methoxyethyl) ether (diglyme). The polar solvent can also be a ketone, such as acetone or acetylacetone. Nitriles, such as, for example, acetonitrile ( ACN), methoxyacetonitrile, propionitrile, butyronitrile, isobutyronitril, benzonitrile, and 3-methoxypropionitrile can be employed as the solvent, as can amines, such as ethylenediamine and pyridines, or amides such as formamide, n-methylacetamide, N,N-dimethylformamide (DMF), N,N-dimethylacetamide (DMA), and N-methyl-2-pyrrolidinone (NMP). The solvent can also be a carbonate. Non-limiting examples of carbonates include propylene carbonate (PC), ethylene carbonate (EC), dimethyl carbonate (DMC), diethyl carbonate (DEC), ethyl methyl carbonate (EMC), and 1,2-butylene carbonate. The solvent can also be a cyclic ester such as γ-butyrolactone (γ-BL) or γ-valerolactone (γ-VL). Other organic based solvents are also contemplated, such as, for example, hexane, benzene, toluene, nitromethane, nitrobenzene, 1,2-dichloroethane, dimethyl sulfoxide (DMSO), ethyl acetate, and nitroethane to name a few. The solvent may also employ a combination of any of the foregoing solvents.

The DMTD metal salt can be included in the polar solvent at from about 1 to about 50 wt.%, or in some instances, from about 2.5 to about 30 wt.%, or even from about 5 to about 25 wt.% or about 10 to about 20 wt.%.

The electrolyte can also include a supporting redox active species, which may be present at from about 0.1 to about 20 wt.% of the electrolyte, or from about 0.25 to about 15 wt.%, or even from about 0.5 to about 10 wt.%. Such supporting redox active species may be present to make the solution more redox active. Supporting redox active species currently envisaged can include, but are not limited to, monoalkylated DMTD derivatives of Formula A: 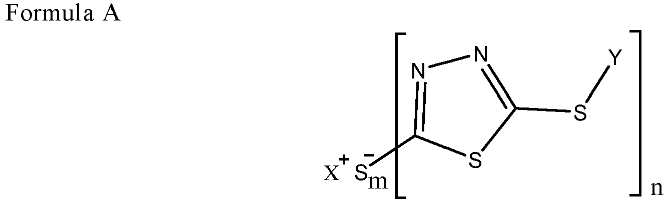 where
"X" is an alkali or alkaline earth metal, such as Li, Na, K, Mg, or Ca, a trialkyl amine, or a quaternary amine, or H;
"m" is 1, 2 or 3, and "n" is 1 or 2; and
where "Y" is:
"[RO]ₒR," where "o" is an integer from 1 to 100;
"R[OH]ₚA," where "p" is an integer from 1 to 6 and "A" is H or an amine, such as a trialkyl amine or a quaternary amine salt;
a carboxamide, such as a (meth)acrylamide of "CH₂CH[CH₃ or H]C(O)NHZ," where "Z" is H, "R," "RSO₃⁻Na⁺," "RSO₃H," or "RN⁺(R)₃Cl⁻," or
a carboxylate, such as an itaconate, maleate, or, for example, a (meth)acrylate of "CH₂CH[CH₃ or H]C(O)OZ," where "Z" can be X, RN⁺(R)₃SO₃⁻, RN⁺(R)₃SO₃⁻K⁺, "RSO₃⁻Na⁺," RSO₃⁻NH₄⁺, "RSO₃H," or "RN⁺(R)₃Cl⁻," and
where "R" is a linear, branched, saturated or unsaturated, or cyclic C₁ to C₁₂, or C₁ to C₁₀, or C₁ to C₈, or even C₁ to C₆ alkyl or aryl group, or H.

Generally, the supporting compounds will contain at least one or more carboxylic acids, one or more carboxylate salts, or at least one carboxamide or ester with a quaternary nitrogen, or sulfonate, or zwitterionic group and combinations thereof to impart water solubility to the compound.

Examples of the R group in Formula A can include methyl, ethyl, propyl, butyl, pentyl, hexyl, heptyl, octyl, nonyl, decyl, undecyl, and dodecyl groups, including branched versions thereof, such as, for example, isobutyl, ethylhexyl, isoamyl, and the like, as well as aromatic groups (e.g. benzyl, phenyl, tolyl, xylyl, naphthyl, etc.) or cyclic groups (e.g., cyclohexyl).

In preparing the monoalkylated DMTD derivatives, a base may be employed. Such a base may be, for example, an alkaline or alkaline earth metal hydroxide or carbonate, or an amine, such as, for example a trialkyl amine. If a base is employed, "X" in the foregoing formulas can be an alkali or alkaline earth metal such as Li, Na, K, Mg, or Ca, or a trialkyl amine. If a base is not employed, "X" would be H and the positive and negative charges associated therewith would be absent.

In its un-coupled form, m and n in Formula A would both be 1, as shown in Formula B below.

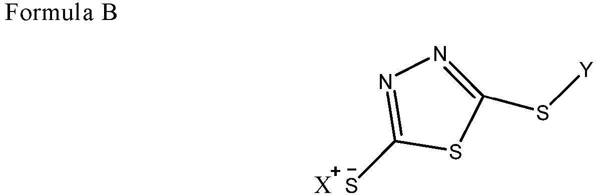

The reaction products can also be further reacted with an oxidizing agent, such as, for example, hydrogen peroxide, to form a sulfur coupled *"bis-"* form of the particular derivative. In the case where the DMTD derivative is sulfur coupled, "n" in Formula A would be 2, "m" could be 1, 2 or 3, and "X" and the positive and negative charges associated therewith would be absent. Such coupled monoalkylated DMTD derivatives can be prepared by preparing the un-coupled compound and then introducing an oxidizing reagent, such as hydrogen peroxide or others, as would be known by those of ordinary skill in the art. Examples of coupled DMTD derivative are shown in Formulas C, D and E below.

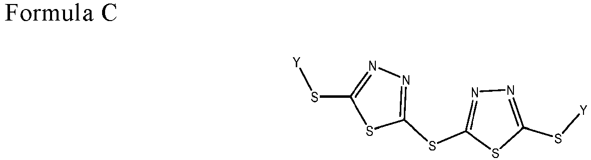

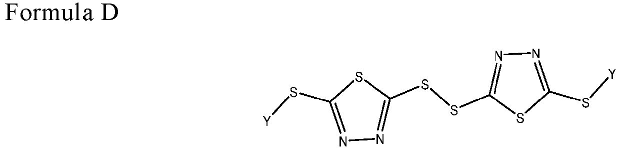

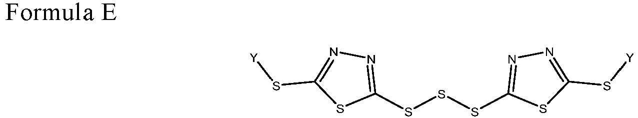

As used herein, the term "group," for example as in a halogenated alkyl or aryl group, (meth)acrylate group, and (meth)acrylamide group, depending on the context, refers to the structure of the stated group on its own or as the structure in which the group would form after reaction with another compound. For example, a methyl group could be CH₄, as in its lone state, or -CH₃ as in its bonded form, or, as a further example, methyl acrylate, could refer to CH₂=CHC(=O)OCH₃, as in its lone state, or -CH₂CH₂C(O)OCH₃, as in its bonded form.

The monoalkylated DMTD derivatives can also be the reaction product resulting from a 1,4 addition between DMTD and a carboxamide group capable of 1,4 addition. The foregoing reaction product can further be reacted in an acid-base reaction with a base to form a salt or an oxidizing agent to form a *bis-* compound.

Carboxamide groups capable of 1,4 addition can be readily envisaged by those of skill in the art, and include, both mono-carboxamides and di-carboxamides. Example carboxamides can include, but not be limited to, itaconic amide, citraconic amide, maleic amide, fumaric amide, mesaconic amide, as well as (meth)acrylamide. The carboxamides can be primary, or can be substituted with one or more "R" groups to form a secondary or tertiary amide group.

As used herein, the parentheses "()" around the term "meth" means the term "meth" may or may not be present. Thus, (meth)acrylate can refer to both acrylate and methacrylate, and (meth)acrylamide includes both acrylamide and methacrylamide.

In a particular example, carboxamide containing monoalkylated DMTD derivatives can result from 1,4 addition between DMTD and a (meth)acrylamide group, wherein the substituent Y of the monoalkylated DMTD derivatives of Formula A would be "CH₂CH[CH₃ or H]C(O)NHZ," and Z is as defined above. Such mono-(meth)acrylamide DMTD derivatives may be represented by Formula H: 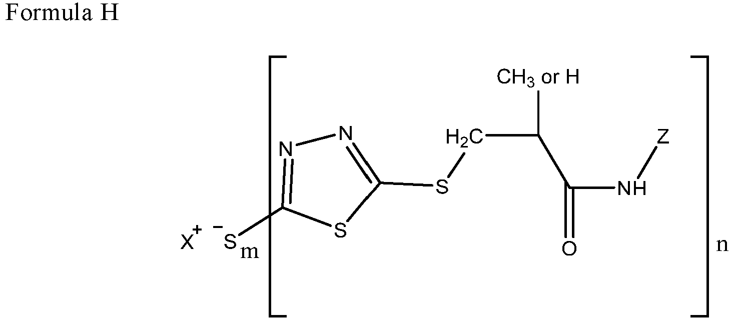 where X, m, and n are as set forth for Formula A, and "Z" is H, "R," RSO₃⁻Na⁺, RSO₃H, or RN⁺(R)₃Cl⁻.

Where the mono-(meth)acrylamide DMTD derivative is not reacted with a base, the mono-(meth)acrylamide DMTD derivative would be represented by:

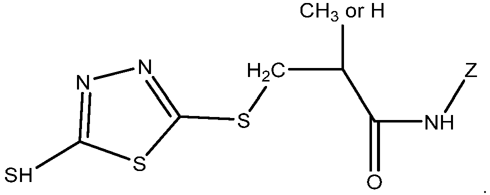

Where the mono-(meth)acrylamide DMTD derivative is reacted with a base, the mono-(meth)acrylamide DMTD derivative would be represented by:

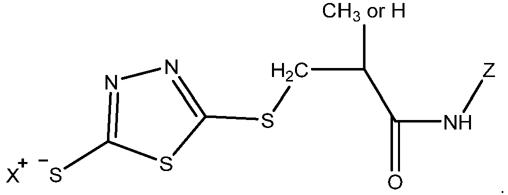

Where the mono-(meth)acrylamide DMTD derivative is reacted with an oxidizing reagent, the mono-(meth)acrylamide DMTD derivative would be represented by:

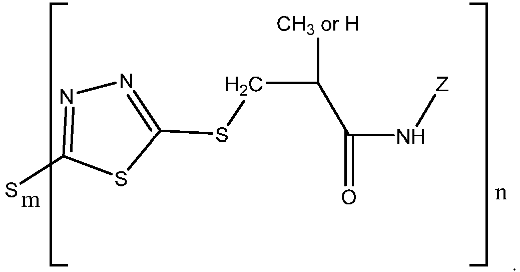

In some embodiments, DMTD can be reacted with methacrylaminopropyl trimethyl ammonium chloride to provide a mono-methacrylamide DMTD derivative of Formula H, in either coupled or un-coupled form, where Z (CH₂)₃N⁺(CH₃)₃Cl⁻, m is 1, 2 or 3 and n is 1 or 2 and X is an optional alkali or alkaline earth metal or amine.

In some embodiments, DMTD can be reacted with 2-acrylamino-2-methylpropane sulfonic acid to provide a DMTD derivative of Formula H, in either coupled or un-coupled form, where Z is C(CH₃)₂CH₂SO₃H, m is 1, 2 or 3 and n is 1 or 2 and X is an optional alkali or alkaline earth metal or amine.

In some embodiments, DMTD can be reacted with 2-acrylamino-2-methylpropane sulfonic acid sodium salt to provide a DMTD derivative of Formula H, in either coupled or un-coupled form, where Z is C(CH₃)₂CH₂SO₃⁻Na⁺, m is 1, 2 or 3 and n is 1 or 2 and X is an optional alkali or alkaline earth metal or amine.

Likewise, the monoalkylated DMTD derivatives can also be the reaction product resulting from a 1,4 addition between DMTD and a carboxylate group capable of 1,4 addition. The foregoing reaction product can further be reacted in an acid-base reaction with a base to form a salt or an oxidizing agent to form a *bis-*compound.

Carboxylate groups capable of 1,4 addition can be readily envisaged by those of skill in the art, and include, both mono-carboxylates and di-carboxylates. Example carboxylates can include, but not be limited to, itaconates, citraconates, maleates, fumarates, mesaconates, as well as (meth)acrylates. The carboxylates can be in the form of a salt with an alkali or alkaline earth metal, or an ester with an "R" group. Where the salt is desired, it can be obtained by 1,4 addition of the salted monomer, or by addition of the ester followed by saponification with an alkali or alkaline earth metal hydroxide. In some embodiments, the carboxylate does not include a (meth)acrylic acid or a (meth)acrylate where the ester group of the (meth)acrylate is a simple R group.

For example, a carboxylate containing DMTD derivative can result from 1,4 addition between DMTD and a dialkyl itaconate, such as dimethyl itaconate, wherein the substituent Y of the monoalkylated DMTD derivatives of Formula A would be dimethyl itaconate, for example, as represented by formula

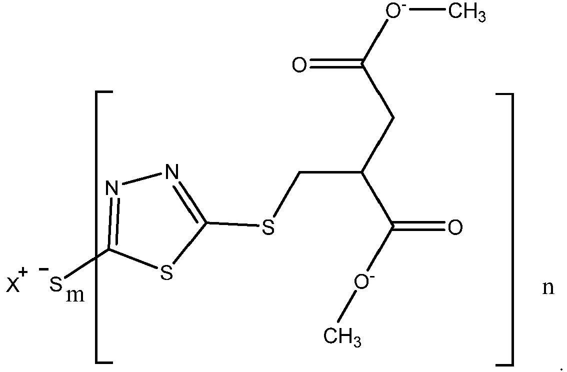

In the foregoing example, the carboxylate could also be, for example, a dialkali or dialkaline earth itaconate salt, such disodium itaconate, represented, for example, by formula

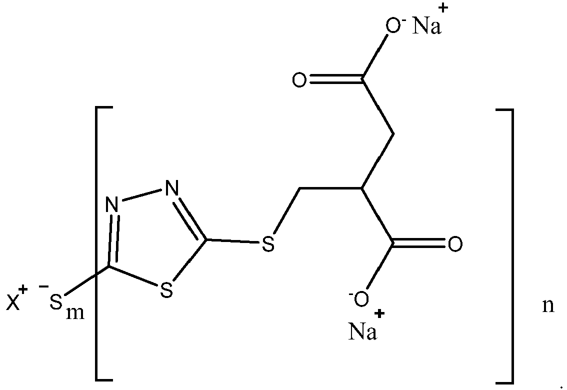

Another example carboxylate containing DMTD derivative can result from 1,4 addition between DMTD and a dialkyl maleate, such as dimethyl maleate, wherein the substituent Y of the monoalkylated DMTD derivatives of Formula A would be dimethyl maleate, for example, as represented by formula

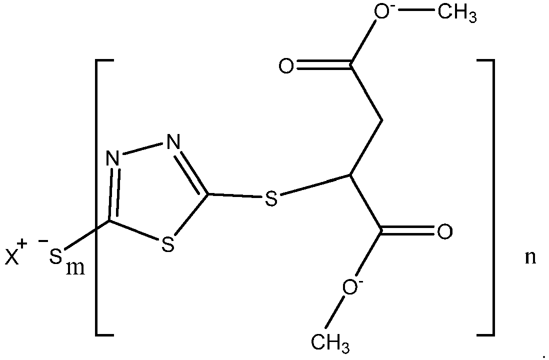

In the foregoing example, the carboxylate could also be, for example, a dialkali or dialkaline earth maleate salt, such disodium maleate, represented, for example, by formula

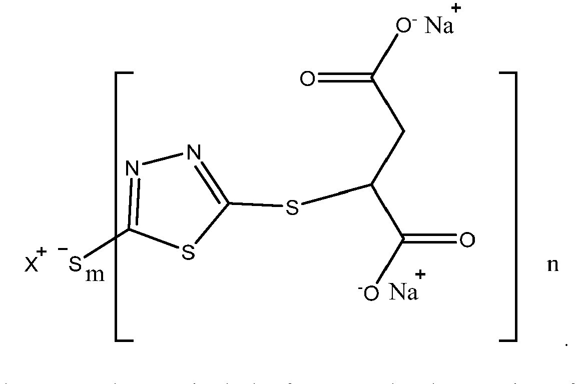

Further examples can include, for example, the reaction of methyl acrylate and DMTD, or dimethyl maleate and DMTD. Where the salts are desired, these examples can also include, for example, the reaction of sodium acrylate or disodium maleate, or the foregoing esters could be saponified in the presence of sodium hydroxide to obtain the salts.

In further examples, a carboxylate containing monoalkylated DMTD derivatives can result from 1,4 addition between DMTD and a (meth)acrylate group in which the ester group is a quaternary ammonium group or a sulfur trioxide containing group, wherein the substituent Y of the monoalkylated DMTD derivatives of Formula A would be "CH₂CH[CH₃ or H]C(O)OZ." The mono-(meth)acrylate DMTD derivatives may be represented by Formula J: 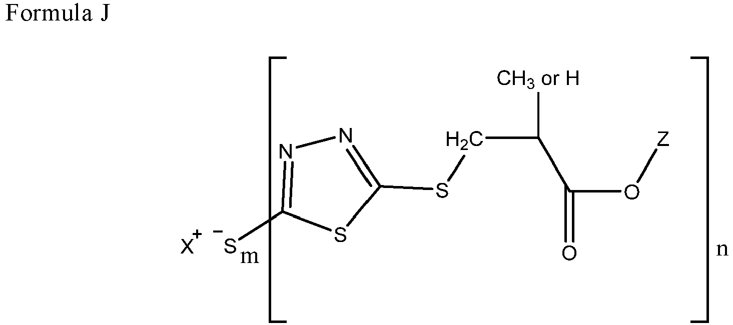 where X, m, and n are as set forth for Formula A, and "Z" is X, RSO₃⁻Na⁺, RSO₃H, RN⁺(R)₃SO₃⁻, RN⁺(R)₃SO₃⁻K⁺, RSO₃⁻NH₄⁺ or RN⁺(R)₃Cl⁻.

Where the mono-(meth)acrylate DMTD derivative is not reacted with a base, the mono-(meth)acrylate DMTD derivative would be represented by: 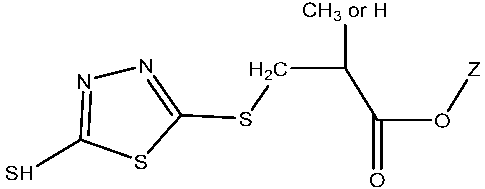 where Z is X, RSO₃⁻Na⁺, RSO₃H, RN⁺(R)₃SO₃⁻, RN⁺(R)₃SO₃⁻K⁺, RSO₃⁻NH₄⁺ or RN⁺(R)₃Cl⁻.

Where the mono-(meth)acrylate DMTD derivative is reacted with a base, the mono-(meth)acrylate DMTD derivative would be represented by: 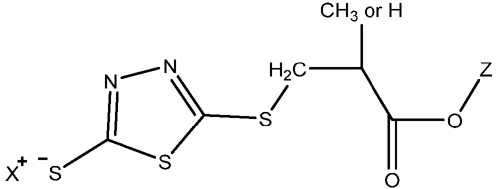 where Z is X, RSO₃⁻Na⁺, RSO₃H, RN⁺(R)₃SO₃⁻, RN⁺(R)₃SO₃⁻K⁺, RSO₃⁻NH₄⁺ or RN⁺(R)₃Cl⁻.

Where the mono-(meth)acrylate DMTD derivative is reacted with an oxidizing reagent, the mono-(meth)acrylate DMTD derivative would be represented by: 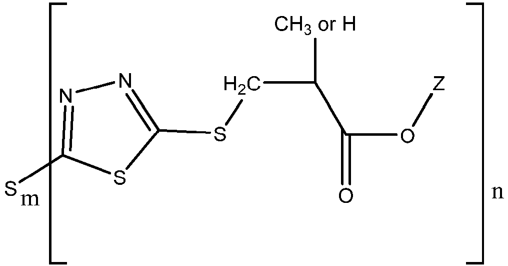 where Z is X, RSO₃⁻Na⁺, RSO₃H, RN⁺(R)₃SO₃⁻, RN⁺(R)₃SO₃⁻K⁺, RSO₃⁻NH₄⁺ or RN⁺(R)₃Cl⁻.

In an example embodiment, DMTD can be reacted with methacryloxyethyl trimethyl ammonium chloride to provide a mono-methacrylate DMTD derivative of Formula J, in either coupled or un-coupled form, where Z is (CH₂)₂N⁺(CH₃)₃Cl⁻, m is 1, 2 or 3 and n is 1 or 2 and X is an optional alkali or alkaline earth metal or amine.

Likewise, the monoalkylated DMTD derivatives can also be the reaction product resulting from a 1,4 addition between DMTD and a (meth)acrylate group and an oxidizing agent to form a *bis-* compound.

In *bis*-(meth)acrylate containing monoalkylated DMTD derivatives resulting from 1,4 addition between DMTD and a (meth)acrylate group and an oxidizing agent, the substituent Y of the monoalkylated DMTD derivatives of Formula A would be "CH₂CH[CH₃ or H]C(O)OZ," and Z is R or H. The mono-(meth)acrylate DMTD derivatives may be represented by Formula K: 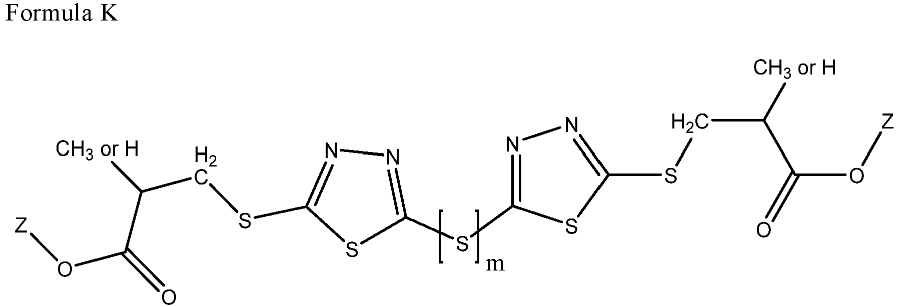 where m is 1, 2 or 3 and "Z" is R or H.

In an example embodiment, DMTD can be reacted with methyl acrylate, followed by an oxidizing agent to provide mono-acrylate bis-DMTD derivative of Formula K, where Z is CH₃, and m is 1, 2 or 3.

In another example embodiment, DMTD can be reacted with 2-ethylhexyl acrylate, followed by an oxidizing agent, to provide a mono-acrylate bis-DMTD derivative of Formula K where Z is a 2-ethylhexyl group, and m is 1, 2 or 3.

The monoalkylated DMTD derivatives can also be the reaction product resulting from the displacement using electron deficient hydrocarbons between DMTD and a halogenated hydrocarbyl ("halo-hydrocarbyl") group (which include linear, branched, saturated or unsaturated, or cyclic alkyl group, or aryl group, where an unsaturated alkyl group is synonymous with an alkene), and further reacted with an oxidizing agent to form a *bis-* compound.

In the monoalkylated DMTD derivatives resulting from the displacement using electron deficient hydrocarbons between DMTD and a halo-hydrocarbyl group, the substituent Y of the monoalkylated DMTD derivatives of Formula A would be "R," as defined above, n would be 2 and m would be 1, 2 or 3. Although the substituent may be any hydrocarbyl group, the derivative is collectively referred to herein and in the claims as a "mono-alkylated" DMTD derivative.

The mono-alkylated *bis*-DMTD derivatives may be represented by Formula L: 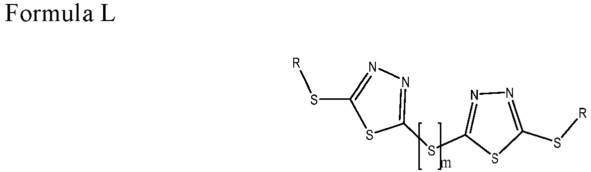 where m is 1, 2 or 3, and R is a linear, branched, saturated or unsaturated, cyclic C₁ to C₁₂, or C₁ to C₁₀, or C₁ to C₈, or even C₁ to C₆ alkyl or aryl group; or H.

In an example embodiment, DMTD can be reacted with a benzylhalide and an oxidizing reagent, to provide a mono-arylated DMTD derivative of one or more of Formula L(1), (2), or (3).

| L(1) |
|---|
| 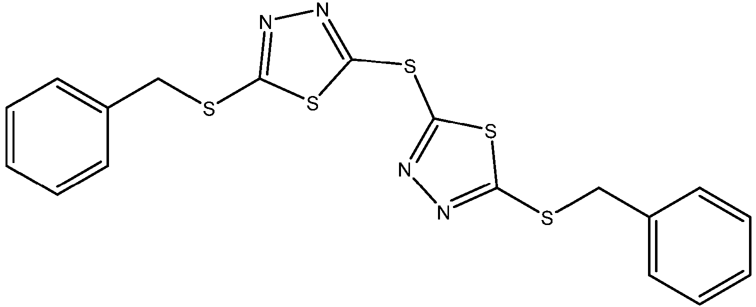 |

| L(2) |
|---|
| 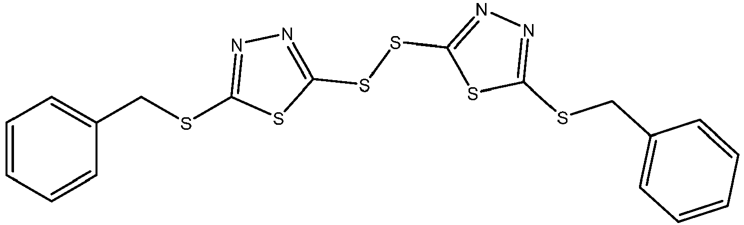 |

| L(3) |
|---|
| 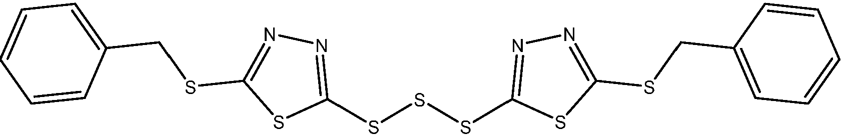 |

In some embodiments, monoalkylated DMTD derivatives such as those discussed above may include a quaternary ammonium substituent and be treated with a strong base, such as LiOH, NaOH, KOH, Ca(OH)₂, a quaternary amine such as R₄NOH, and the like to produce a zwitterionic species.

The foregoing reaction products can include a zwitterionic mono-quaternary ammonium containing alkyl DMTD derivative represented as shown in formula M:

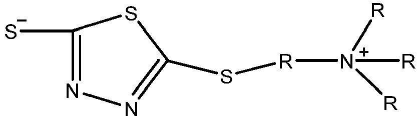

The foregoing reaction products can include a zwitterionic mono-quaternary ammonium containing ether DMTD derivative represented as shown in formula N:

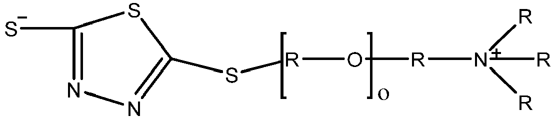

The foregoing reaction products can also include a zwitterionic mono-quaternary ammonium containing alcohol DMTD derivative represented as shown in formula O:

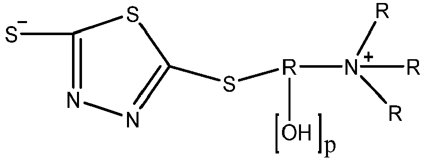

The foregoing reaction products can include a zwitterionic mono-quaternary ammonium containing carboxamide, such as, for example, a (meth)acrylamide DMTD derivative represented as shown in formula P:

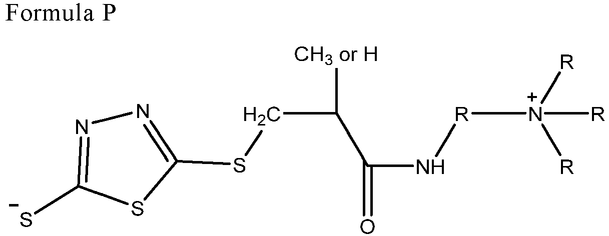

The foregoing reaction products can include a zwitterionic mono-quaternary ammonium containing carboxylate, such as, for example, a (meth)acrylate DMTD derivative represented as shown in formula Q:

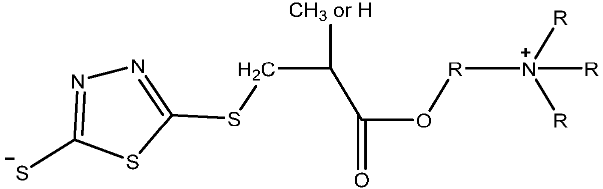

For example, DMTD can be reacted with methacrylaminopropyl trimethyl ammonium chloride to provide a mono-methacrylamide DMTD derivative of Formula XV where Z (CH₂)₃N⁺(CH₃)₃Cl⁻, m is 1, 2 or 3 and n is 1 or 2, followed by treatment with NaOH to afford a zwitterion of formula R:

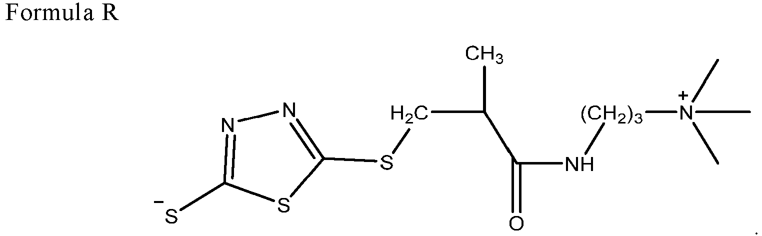

The foregoing monoalkylated DMTD derivatives can be included as supporting redox active species at up to 1 mole equivalent with the DMTD metal salt derivatives disclosed herein. In some cases, the monoalkylated DMTD derivatives can be included as supporting redox active species from 0.25 up to 1 mole equivalent with the DMTD metal salt derivatives disclosed herein. In some cases, the monoalkylated DMTD derivatives can be included as supporting redox active species from 0.5 up to 1 mole equivalent with the DMTD metal salt derivatives disclosed herein.

Further supporting redox active species currently envisaged include, but are not limited to, lithium salts, sodium salts, potassium salts, ammonium salts, and mixtures thereof. Examples of supporting redox active species include, but are not limited to, for example, sodium phosphate, sodium chloride, potassium phosphate, potassium chloride, potassium hexafluorophosphate, lithium hexafluorophosphate, tetrabutylammonium fluorophosphate, ammonium chloride, tetrabutylammonium chloride, lithium perchlorate, lithium nitrate, lithium chloride, and the like.

The foregoing electrolyte may further include acids, bases, supporting electrolytes, additives, including redox mediators such as metallocenes and viologens, co-solvents, emulsifiers, other redox active compounds, ionic liquids, viscosity-controlling agents, wetting agents, stabilizers, salts, or combinations thereof.

In particular, the foregoing electrolyte may include buffering agents. In an embodiment, the electrolyte can have a pH of between 6 to 8. In some instance, the electrolyte can have a pH of between 6.25 and 7.75. The electrolyte can also have a pH of between 6.5 and 7.5. In some embodiments, the electrolyte can have a pH of between 6.75 and 7.25. In further embodiments, the electrolyte can have a pH of between 6.8 and 7.2, or 6.9 and 7.1, or even 6.95 and 7.05. The pH can be adjusted by adding buffering agents, such as, for example, potassium phosphate, dipotassium phosphate, tripotassium phosphate, and other typical buffers, such as sulfamic acids/sulfamates, formic acids/formates, acetic acids/acetates, ammoniums, bicarbonates/ carbonates, fumaric acids/hydrogen fumarates, benzoic acids/benzoates, and the like.

Also provided is a redox flow battery system including the above discussed electrolyte. A typical RFB comprises a positive half-cell and a negative half-cell. The positive half-cell comprises an electrode tank containing a catholyte and the negative half-cell comprises an electrode tank containing an anolyte. The anolyte and catholyte are solutions comprising electrochemically active components in different oxidation states. The anolyte and catholyte are often referred to as the negative electrolyte and positive electrolyte, respectively, and these terms can be used interchangeably. As used herein, the terms anolyte and catholyte refer to electrolytes composed of the electrochemically active components and an aqueous supporting solution.

The RFB system provided herein will include a half-cell containing an electrolyte containing the DMTD metal salts discussed above either alone, or in combination with a monoalkylated DMTD derivative. In the battery system, it is postulated that the DMTD metal salt, during a charging state, form A) a coupled compound, which can be referred to as DSSD, two charged compounds, which can be referred to as 2DS⁻, or a mixture thereof in the polar solvent, and B) a coupled compound DSSD or 2S⁻, or a mixture thereof in a polar solvent, where D is the main DMTD body, e.g.: 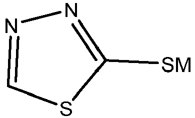 and S is from the salted sulfur off the main DMTD body, e.g.: 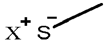 so that DSSD refers to, for example: 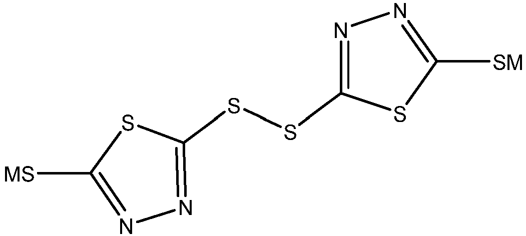 and DS⁻ refers to, for example: 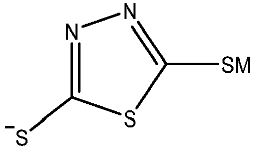 where Y is as described above.

The addition of monoalkylated DMTD derivatives can further decrease the charge-discharge gaps and increase the capacity of the DMTD metal salt containing electrolyte.

The RFB system provided herein will also include a separate half-cell containing a second electrolyte with a different redox active species other than the DMTD metal salts and monoalkylated DMTD derivatives discussed above. The selection of aqueous electrolytes and redox active species is generally based on their electrochemical properties (e.g., stability window), physical properties (e.g., viscosity, vapor properties), safety (e.g., corrosiveness, toxicity), and cost. Possible candidate electrolytes include acidic solutions (e.g., H₂SO₄, CH₃SO₃H), mild to neutral pH solutions containing alkali-ion salts (e.g., Li₂SO₄, KCl), or basic solutions (e.g., LiOH, KOH). The selection of redox active species is based on their electrochemical properties (redox potential, reversibility, etc.), physical properties (solubility, etc.), safety, and cost. Possible redox active species include organic molecules with unsaturated moieties and/or conjugated unsaturated moieties. These moieties can be aromatic, non-aromatic, or a combination thereof, and comprise carbon-carbon unsaturated bonds, carbon-heteroatom unsaturated bonds, or a combination of carbon-carbon and carbon-heteroatom unsaturated bonds, and wherein the heteroatom is a non-metallic heteroatom or a metalloid heteroatom.

In certain embodiments, the additional type of redox active species can include, for example, a metal, metal salt, or metal-ligand coordination compound. In certain embodiments, the additional type of redox active species can include vanadium. In certain embodiments, the additional type of redox active species can include iron. In certain embodiments, the additional type of redox active species can include chromium. In certain embodiments, the additional type of redox active species can include manganese. In certain embodiments, the additional type of redox active species can include titanium. In certain embodiments, the additional type of redox active species can include zinc. In certain embodiments, the additional type of redox active species can include an inorganic compound. In certain embodiments, the additional type of redox active species can include bromine. In certain embodiments, the additional type of redox active species can include an organic compound. In certain embodiments, the additional type of redox active species can include a hexacyanoiron complex, a quinone, a hydroquinone, or an organic dye, or a salt or mixture thereof. In certain embodiments, the additional type of redox active species can include a hexacyanoiron complex. In certain embodiments, the additional type of redox active species can include ferrocyanide. In certain embodiments, the additional type of redox active species can include ferricyanide. In certain embodiments, the additional type of redox active species can include a quinone, hydroquinone, sulfonated quinone, or sulfonated hydroquinone. In certain embodiments, the additional type of redox active species can include 1,2-dihydroxy-3,5-benzene disulfonate (Tiron). In certain embodiments, the additional type of redox active species can include 1,2,4-trihydroxy-3,5-benzenedisulfonate. In certain embodiments, the additional type of redox active species can include an anthraquinone or sulfonated anthraquinone. In certain embodiments, the additional type of redox active species can include 9,10-anthraquinone. In certain embodiments, the additional type of redox active species can include 9,10-anthraquinone-2,7-disulphonic acid. In certain embodiments, the additional type of redox active species can include an organic dye. In certain embodiments, the additional type of redox active species can include indigo carmine. In certain embodiments, the additional type of redox active species can include a viologen. In certain embodiments, the additional type of redox active species can include methyl viologen or benzylviologen. In certain embodiments, the additional type of redox active species can include a tetrazole, diaryl ketone, dipyridyl ketone, dialkoxy benzene, phenothiazine, catechol, catechol ether, or catechol phenylborate ester. In certain embodiments, the additional type of redox active species can include di-(2-pyridyl)-ketone, 2,5-di-tert-butyl-1,4-bis(2-methoxyethoxy)benzene (DBBB), 2,5-di-tert-butyl-1,4-dimethoxybenzene, 2,5-di-tert-butyl-1,4-bis(2,2,2-trifluoroethoxy)benzene, phenothiazine, catechol, tetrafluorocatechol, or 5,6,7,8-tetrafluoro-2,3-dihydrobenzodioxine. In certain embodiments, the additional type of redox active species can include 5-mercapto-1-methyltetrazole.

The half-cells in the RFB will be separated by a membrane or separator, such as an ion-exchange membrane (cation- or anion-exchange membrane), ion conductive membrane (polymer or ceramic) or porous separator, through which the electrolyte can readily flow. Current membranes envisaged for the RFB system include, but are not limited to, sulfonated tetrafluouroethylene based fluoropolymer-copolymer membranes, for example, cellulose-based dialysis membranes, and membranes of functionalized polystyrene blended with polyvinyl chloride. However, any membrane that can prevent crossover of the redox species in both states of charge, and allow diffusion of either positive or negative ions as charge carriers to maintain charge neutrality can be employed.

In some embodiments, at least one of the catholyte and anolyte redox active materials can remain fully soluble during the charging and discharging cycles of the RFB. However, one member of a redox pair may be insoluble or partially soluble during the charging and discharging cycles of the RFB. For example, when the anolyte comprises a metal cation/metal atom redox pair, only the cation can remain fully soluble during the charging and discharging cycles.

During charging and discharging of the RFB, the catholyte and anolyte are continuously circulating via pumps through the positive and negative electrodes respectively, where redox reactions proceed, providing the conversion between chemical energy and electrical energy or vice-versa. To complete the circuit during use, positive and negative electrodes (including a current collector at each side) of the RFB system are electrically connected through current collectors with an external load.

The amount of each chemical component described is presented exclusive of any solvent or diluent oil, which may be customarily present in the commercial material, that is, on an active chemical basis, unless otherwise indicated. However, unless otherwise indicated, each chemical or composition referred to herein should be interpreted as being a commercial grade material which may contain the isomers, byproducts, derivatives, and other such materials which are normally understood to be present in the commercial grade.

### EXAMPLES

**Example 1.** Synthesis of Dipotassium 1,3,4-thiadiazole-2,5-dithiolate. 40g of 2,5-dimercapto-1,3,4-thiadiazole and 232g of deionized water were added to a 1-L flask and 29.9g of potassium hydroxide was added over 15 minutes. The reaction was heated at 75 °C and held there for five hours. The clear product solution was cooled and transferred to a storage bottle. The product yield was 299.9g.

**Example 2.** Synthesis of Dipotassium 1,3,4-thiadiazole-2,5-dithiolate. 125g of 2,5-dimercapto-1,3,4-thiadiazole and 330g of reagent grade methanol were added to a 2-L flask and cooled to 10 °C in an ice-water bath. 186.73g of potassium t-butoxide was added over 60 minutes with stirring under nitrogen while keeping the reaction temperature under 20 °C. The reaction was slowly heated to reflux at 63 °C and held there for six hours. After transferring to an open beaker, most of the methanol was evaporated in a steam oven. The crude product was dried in a vacuum oven at 100 °C to constant weight. The product yield was 185.3g.

**Example 3.** Synthesis of Disodium 1,3,4-thiadiazole-2,5-dithiolate. 127.7g of 2,5-dimercapto-1,3,4-thiadiazole and 550g of reagent grade ethanol were added to a 2-L flask and cooled to 10 °C in an ice-water bath. 91.85g of sodium methoxide was added over 60 minutes with stirring under nitrogen while keeping the reaction temperature under 30 °C. The reaction was slowly heated to reflux at 72 °C and held there for four hours. After transferring to an open beaker, most of the ethanol was evaporated in a steam oven. The crude product was dried in a vacuum oven at 100 °C to constant weight. The product yield was 162g.

**Example 4.** Synthesis of Dilithium 1,3,4-thiadiazole-2,5-dithiolate. 46g of 2,5-dimercapto-1,3,4-thiadiazole and 400g of reagent grade methanol were added to a 2-L flask and cooled to 10 °C in an ice-water bath. 59.67g of lithium t-butoxide was added over 20 minutes with stirring under nitrogen while keeping the reaction temperature under 20 °C. The reaction was slowly heated to reflux at 62 °C and held there for five hours. After transferring to an open beaker, most of the methanol was evaporated in a steam oven. The product was dried in a vacuum oven over two days at 120 °C to constant weight. The product yield was 59.2g.

**Example 5.** Synthesis of Potassium 1,3,4-thiadiazole-2-thiol-5-thiolate. 300g of reagent grade methanol and 89.63g of potassium t-butoxide were added to a 2-L flask and cooled to 5 °C in an ice-water bath. 120g of 2,5-dimercapto-1,3,4-thiadiazole was added in portions over 45 minutes while keeping the reaction temperature under 15 °C. The reaction was slowly heated to reflux at 62 °C and held there for six hours. After transferring to an open beaker, most of the methanol was evaporated in a steam oven. The product was dried in a vacuum oven at 120 °C to constant weight. The product yield was 148.7g.

**Example 6.** Synthesis of Sodium 1,3,4-thiadiazole-2-thiol-5-thiolate. 120g of 2,5-dimercapto-1,3,4-thiadiazole and 320g of reagent grade methanol were added to a 2-L flask and cooled to 10 °C in an ice-water bath. 43.13g of sodium methoxide was added over 30 minutes with stirring under nitrogen while keeping the reaction temperature under 20 °C. The reaction was slowly heated to reflux at 63 °C and held there for six hours. After transferring to an open beaker, most of the methanol was evaporated in a steam oven. The product was dried in a vacuum oven at 120 °C to constant weight. The product yield was 136g.

**Example 7.** Synthesis of Lithium 1,3,4-thiadiazole-2-thiol-5-thiolate. 350g of reagent grade methanol and 41.6g of lithium t-butoxide under nitrogen were added to a 1-L flask and cooled to 5 °C in an ice-water bath. 78.1g of 2,5-dimercapto-1,3,4-thiadiazole was added in portions over 45 minutes while keeping the reaction temperature under 15 °C. The reaction was slowly heated to 60 °C and held there for three hours. After transferring to an open beaker, most of the solvent was evaporated in a steam oven. The product was dried in a vacuum oven at 140 °C to constant weight. The product yield was 78g.

**Example 8.** Synthesis of 2-(Acryloylamino-2-methylpropylsulfonic acid)thio-5-thiol-1,3,4-thiadiazole. 98.15 grams of DMTD, 135.4 grams of 2-Acrylamido-2-methylpropanesulfonic acid, 0.2 gram of sodium hydroxide, and 200 grams of isopropanol solvent were added to a one liter flask and heated under nitrogen at 80 °C for 8 hours. The mixture was transferred to an open beaker and most of the water solvent was evaporated in a 65 °C oven on standing, followed by vacuum drying at 4 mm Hg and 95 °C for 22 hours. The final product was a crystalline solid.

**Example 9.** Synthesis of Disodium 2-(acryloylamino-2-methylpropylsulfonate)thio-5-thiolate-1,3,4-thiadiazole. 72.7 grams of 2-(acryloylamino-2-methylpropylsulfonic acid)-5-thiol-1,3,4-thiadiazole prepared in Example 8, 16.25 grams of sodium hydroxide, 200 grams of deionized water solvent were added to a one liter flask and heated under nitrogen at 85 °C for 3 hours. The mixture was transferred to an open beaker and most of the water solvent was evaporated in a 65 °C oven on standing, followed by vacuum drying at 3 mm Hg and 105 °C for 16 hours. The final product was a crystalline solid.

**Example 10.** Synthesis of Dipotassium 2-(acryloylamino-2-methylpropylsulfonate)thio-5-thiolate-1,3,4-thiadiazole. A procedure similar to Example 9 was used starting from Example 8 and potassium hydroxide.

**Example 11.** Synthesis of 2-(Methacryloylaminopropyltrimethylammonium chloride)thio-5-thiol-1,3,4-thiadiazole. 84.1 grams of 2,5-dimercapto-1,3,4-thiadiazole, 247 grams of 50% aqueous methacryloylaminopropyltrimethylammonium chloride, 0.42 gram of sodium hydroxide, and 300 grams of deionized water were added to a one liter flask and heated under nitrogen at 92 °C for 8 hours. The mixture was transferred to an open beaker and most of the water solvent was evaporated in a 65 °C oven on standing, followed by vacuum drying at and 90 °C. The final product was a light brown crystalline solid.

**Example 12.** Synthesis of 2-(Methacryloylaminopropyl trimethylammonium)thio-5-thiolate-1,3,4-thiadiazole. 122.7 grams of 2-(methacryloylaminopropyltrimethylammonium chloride) thio-5-thiol-1,3,4-thiadiazole prepared in Example 11, 13.2 grams of sodium hydroxide, and 200 grams of deionized water were added to a one liter flask and heated under nitrogen at 70 °C for three hours. The mixture was transferred to an open beaker and most of the water solvent was evaporated in a 65 °C oven on standing, followed by vacuum drying at 5 mm Hg and 95 °C for 18 hours. The crystalline solid obtained was dissolved in 250 mL of ethanol and cooled to precipitate sodium chloride, which was removed by filtration. Ethanol was removed in a 65 °C oven and a vacuum oven to constant weight at 95 °C.

**Example 13.** Synthesis of 2-(Methacryloyloxyethyl trimethylammonium chloride)thio-5-thiol-1,3,4-thiadiazole. 65.9 grams of 2,5-dimercapto-1,3,4-thiadiazole, 126.5 grams of 72% aqueous methacryloyloxyethyltrimethylammonium chloride, 0.2 gram of sodium hydroxide, and 125 grams of deionized water were added to a one liter flask and heated under nitrogen at 90 °C for 8 hours. The mixture was transferred to an open beaker and most of the water solvent was evaporated in a 65 °C oven on standing, followed by vacuum drying to constant weight at 90 °C. The final product was a crystalline solid.

**Example 14.** Synthesis of 2-(2,3-Dicarboxypropyl)thio-5-thiol-1,3,4-Thiadiazole. 105.16g of 2,5-dimercapto-1,3,4-thiadiazole, 300g of deionized water, 91.1g of itaconic acid and 0.3g of sodium hydroxide catalyst were added to a 1-L flask and heated under nitrogen at 88 °C for 10 hours and cooled. Most of the water was evaporated in a steam oven at 65 °C in an open beaker. The product was then dried in a vac oven at 95 °C until constant weight. The product yield was 194g.

**Examples 15-18.** A procedure similar to Example 14 was used starting from acrylic acid, methacrylic acid, maleic acid, or aconitic acid to prepare the following:
**Example 15.** 2-(2-Carboxyethyl)thio-5-thiol-1,3,4-thiadiazole
**Example 16.** 2-(2-Methylethyl-2-carboxy)thio-5-thiol-1,3,4-thiadiazole
**Example 17.** 2-(2,3-Dicarboxyethyl)thio-5-thiol-1,3,4-thiadiazole
**Example 18.** 2-(1,2,3-Tricarboxypropyl)thio-5-thiol-1,3,4-thiadiazole

**Example 19.** Synthesis of Trisodium 2-(propyl-2,3-dicarboxylate)thio-5-thiolate-1,3,4-thiadiazole. 40g of 1,3,4-thiadiazole-2,5-dithiol-monosodium salt, 300g of deionized water, and 30.2g of itaconic acid were added to a 1-L flask and heated under nitrogen at 85-90 deg C for 12 hrs. After cooling, 18.6g of sodium hydroxide was added and the reaction heated at 75-80 °C for 6 hours. The reaction mixture was transferred to an open beaker and most of the water was evaporated in a steam oven at 65 °C. The product was dried in a vac oven to constant weight at 90 °C to obtain 78.2g of a light yellow solid. ¹³C NMR showed a mixture of the desired product and sodium DMTD salts was obtained.

**Example 20.** Synthesis of Trisodium 2-(propyl-2,3-dicarboxylate)thio-5-thiolate-1,3,4-thiadiazole. An alternate procedure was used to prepare Example 19. 32g of the diacid product prepared in Example 14, 250g of deionized water, and 13.7g of sodium hydroxide were added to a 1-L flask and heated under nitrogen at 85 °C for 6 hours. The mixture was transferred to an open beaker and most of the water evaporated in a steam oven at 65 °C. The product was dried in a vacuum oven at 95 °C to constant weight to obtain 38.8g of a light yellow solid.

**Examples 21-28.** The procedure of Example 20 was used to prepared the following products:
**Example 21.** Dipotassium 2-(ethyl-2-carboxylate)thio-5-thiolate-1,3,4-thiadiazole
**Example 22.** Tripotassium 2-(ethyl-1,2-dicarboxylate)thio-5-thiolate-1,3,4-thiadiazole
**Example 23.** Tripotassium 2-(propyl-2,3-dicarboxylate)thio-5-thiolate-1,3,4-thiadiazole
**Example 24.** Tetrapotassium 2-(propyl-1,2,3-tricarboxylate)thio-5-thiolate-1,3,4-thiadiazole
**Example 25.** Dipotassium 2-(2-methylethyl-2-carboxylate)thio-5-thiolate-1,3,4-thiadiazole
**Example 26.** Tetrasodium 2-(propyl-1,2,3-tricarboxylate)thio-5-thiolate-1,3,4-thiadiazole
**Example 27.** Disodium 2-(ethyl-1,2-dicarboxylate)thio-5-thiol-1,3,4-thiadiazole
**Example 28.** Disodium 2-(propyl-2,3-dicarboxylate)thio-5-thiol-1,3,4-thiadiazole

**Example 29.** Synthesis of Diammonium 2-(propyl-2,3-dicarboxylate)thio-5-thiol-1,3,4-thiadiazole. 40g of the diacid product prepared in Example 14, 19g of aqueous ammonia solution, and 212.8 of deionized water were added to a 1-L flask and stirred under nitrogen at 25 °C for three hours. The reaction mixture was clear and free of solids. 271g of a 20% weight product solution in water was obtained.

**Example 30.** Synthesis of Triammonium 2-(propyl-1,2,3-tricarboxylate)thio-5-thiol-1,3,4-thiadiazole. A procedure similar to Example 29 was used starting from the triacid prepared in Example 18 to prepare this product as a 20.3% weight solution in deionized water.

**Example 31.** Preparation of a pH 7 Phosphate Buffer in 1M Potassium Chloride. 74.55g of potassium chloride, 6.309g of potassium dihydrogen phosphate, and 9.343g of dipotassium hydrogen phosphate were added to a 1-liter volumetric flask. The mixture was diluted to 1.0 liter with deionized water and mixed until homogenous.

**Example 32.** Preparation of a pH 7 Phosphate Buffer in 1M Sodium Chloride. 58.5g of sodium chloride, 5.56g of sodium dihydrogen phosphate, and 7.62g of disodium hydrogen phosphate were added to a 1-liter volumetric flask. The mixture was diluted to 1.0 liter with deionized water and mixed until homogenous.

Electrochemical testing of the formulated DMTD metal salt electrolytes to confirm they work as RFB electrolytes consisted of Cyclic Voltammetry (CV) millivolt peak gap measurements and H-cell cycle testing/specific energy retention measurements.

CV millivolt peak gap measurements involve scanning the electrolyte with a cyclic Voltammogram at a specified temperature and scan rate. The electrolytes in table 1 were scanned at a temperature of 22 °C and a scan rate of 100 mV/s. A CV cathode/anode peak gap of 600 millivolts or less is considered to be a viable result for electrolyte redox reversibility in an RFB application.

H-cell cycle testing/specific energy retention measurements involve galvanostatic discharge-charge of a symmetric battery, where DMDT metal salt was used on both cathodic and anodic chambers in a H-cell under galvanostatic control. The two compartments of the H-cell were separated with a G5 glass frit. The voltage cutoffs were determined based on CV data. This simple H-cell setup allows a direct evaluation of the stability of DMDT metal salts during electrochemical cycling (J. Am. Chem. Soc. 2016, 138, 13230. and J. Am. Chem. Soc. 2017, 139, 2924.). The charge-discharge cycling was performed at 100% state of charge with a rate of 1 C (one hour charging and one hour discharge). Efficiency ratings of 70-85% for the H-cell testing are considered very good performance.

The results of the CV millivolt and H-Cell tests are shown in Table 1. Where indicated, CV and H-Cell evaluations were run in the pH 7 buffered solutions prepared in Examples 31 or 32.

**Table 1**

| Test | Example (CV blend res. no.) | CV | | | H-Cell | | |
|---|---|---|---|---|---|---|---|
| | | [Concentration] Electrolyte(s) | Cathode/Anode Peak Gap | | [Concentration] Electrolyte | Charge-Discharge Voltage Gap | Cycles Fading per cycle |
| | | Solvent | | | Solvent | | |
| | | | | | | | |
| 1 | Example 2 | [1 mM] | 681 mV | | [10 mM] | 400 mV | 9 cycles |
| | | Example 31 | 744 mV _{(to 2^{nd} peak)} | | Example 31 | | 6.4% fading/cycle |
| 2 | Example 2 | [2 mM] | 552 mV | | [20 mM] | | |
| | | Example 31 | 642 mV _{(to 2^{nd} peak)} | | Example 31 | | |
| 3 | Example 2 | [5 mM] | 732 mV | | [50 mM] | 210 mV | 12 cycles |
| | | Example 31 | 842 mV _{(to 2^{nd} peak)} | | Example 31 | | 2.4% fading/cycle |
| 4 | Example 2 | [10 mM] | 750 mV | | [100 mM] | 120 mV | 16 cycles |
| | | Example 31 | 836 mV _{(to 2^{nd} peak)} | | Example 31 | | 0.2% fading/cycle |
| 5 | Example 2 | [50 mM] | Irreversible | | [200 mM] | 100 mV | 20 cycles |
| | | Example 31 | | | Example 31 | | 2% fading/cycle |
| 6 | Example 3 | [10 mM] | 886 mV | | [10 mM] | 570 mV | 10 cycles |
| | | Example 32 | | | Example 32 | | 4.7% fading/cycle |
| 7 | 1 mole Example 2 + 1 mole Example 19 | [5 mM] | 756 mV | | [10 mM] | 334 mV | 30 cycles |
| | | [5 mM] Example 31 | | | Example 31 | | 2.6% fading/cycle |
| 8 | 1 mole Example 2 + 0.5 mole Example 20 | [10 mM] | 1464 mV | | [10 mM] | 350 mV | 9 cycles |
| | | [5 mM] Example 31 | | | Example 31 | | 5.5% fading/cycle |
| 9 | 1 mole Example 2 + 2 moles Example 20 | [5 mM] | 812 mV | | [10 mM] | 400 mV | 5 cycles |
| | | [10 mM] Example 31 | | | Example 31 | | 4.5% fading/cycle |
| 10 | 1 mole Example 2 + 1 mole Example 9 | [5 mM] | 1023 mV | | [10 mM] | 310 mV | 14 cycles |
| | | [5 mM] Example 31 | | | Example 31 | | 3.85% fading/cycle |
| 11 | 1 mole Example 2 + 1 mole Example 12 | [5 mM] | 714 mV | | [10 mM] | 300 mV | 10 cycles |
| | | [5 mM] Example 31 | | | Example 31 | | 4.6% fading/cycle |
| 12 | Example 12 | [10 mM] Example 31 | 900 mV | | not run | | |
| 13 | Example 13 | [10 mM] Example 32 | 900 mV | | [10 mM] Example 32 | 1000 mV | |
| 14 | Example 13 | [10 mM] Example 31 | 800 mV | | not run | | |
| 15 | Example 15 | [10 mM] Example 31 | 1100 mV | | [10 mM] Example 31 | 800 mV | |
| 16 | Example 17 | [10 mM] Example 31 | 900 mV | | [10 mM] Example 31 | 1100 mV | |
| 17 | Example 19 | [10 mM] Example 32 | 1000 mV | | [10 mM] Example 32 | 400-600 mV | |
| 18 | Example 19 | [8 mM] Example 31 | 850 mV | | [10 mM] Example 31 | 200-400 mV | |
| 19 | Example 19 | [8 mM] Example 31 | Irreversible | | not run | | |
| 20 | Example 19 | [8 mM] Example 31 | 850 mV | | [8 mM] Example 31 | 400-600 mV | |
| 21 | Example 19 | [10 mM] 1M NH₄Cl | 850 mV | | [10 mM] 1M NH₄Cl | > 1000 mV | |
| 22 | Example 20 | [10 mM] Example 31 | 603 mV | | [10 mM] Example 31 | 1000 mV | |
| 23 | Example 30 | [10 mM] Example 31 | 668 mV | | not run | | |

The mention of any document is not an admission that such document qualifies as prior art or constitutes the general knowledge of the skilled person in any jurisdiction. Except in the Examples, or where otherwise explicitly indicated, all numerical quantities in this description specifying amounts of materials, reaction conditions, molecular weights, number of carbon atoms, and the like, are to be understood as modified by the word "about." It is to be understood that the upper and lower amount, range, and ratio limits set forth herein may be independently combined. Similarly, the ranges and amounts for each element of the invention can be used together with ranges or amounts for any of the other elements.

As used herein, the transitional term "comprising," which is synonymous with "including," "containing," or "characterized by," is inclusive or open-ended and does not exclude additional, un-recited elements or method steps. However, in each recitation of "comprising" herein, it is intended that the term also encompass, as alternative embodiments, the phrases "consisting essentially of" and "consisting of," where "consisting of" excludes any element or step not specified and "consisting essentially of" permits the inclusion of additional un-recited elements or steps that do not materially affect the essential or basic and novel characteristics of the composition or method under consideration.

While certain representative embodiments and details have been shown for the purpose of illustrating the subject invention, it will be apparent to those skilled in this art that various changes and modifications can be made therein without departing from the scope of the subject invention. In this regard, the scope of the invention is to be limited only by the appended claims.

## Claims

1. A redox flow battery electrolyte comprising A) a polar solvent, and B) a DMTD metal salt derivative of formula (I): 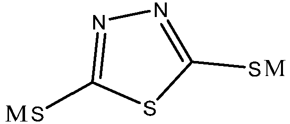 where M is H or an alkali or alkaline earth metal, such as Li, Na, K, Mg, or Ca, with the proviso that at least one of M is an alkaline or alkaline earth metal.

2. The redox flow battery electrolyte of claim 1, wherein both M are an alkaline earth metal.

3. The redox flow battery electrolyte of claim 1, wherein the polar solvent comprises water.

4. The redox flow battery electrolyte of any previous claim, wherein the polar solvent comprises acetonitrile.

5. The redox flow battery electrolyte of any previous claim, wherein the polar solvent comprises an organic solvent, such as carbonates, ethers, ketones, nitriles, alcohols, glycols, amines, amides, organic solvents and combinations thereof.

6. The redox flow battery electrolyte of any previous claim, wherein the DMTD metal salt derivative thereof is present at from about 1 to about 50 wt.% of the electrolyte composition.

7. The redox flow battery electrolyte of any previous claim, further comprising a supporting redox active species containing at least one water soluble mono-alkylated DMTD derivative of formula I.

8. The redox flow battery electrolyte of any previous claim, further comprising a supporting redox active species comprising at least one of a lithium salt, potassium salt, sodium salt, ammonium salt, and combinations thereof.

9. The redox flow battery electrolyte of claim 7 or 8, wherein the supporting redox active species is present at from about 0.1 to about 20 wt.% of the electrolyte composition.

10. A redox flow battery system comprising A) a catholyte comprising the electrolyte of any of claims 1 to 9, and B) an anolyte comprising a redox active species other than the DMTD metal salt derivative, or a mixture thereof in a polar solvent.

11. The redox flow battery system of claim 10 further comprising an ion exchange membrane or microporous membrane separating the anolyte and catholyte.

12. The redox flow battery system of claim 11 where the membrane is a sulfonated tetrafluouroethylene based fluoropolymer-copolymer.

13. The redox flow battery system of claim 11 where the membrane is a cellulose-based dialysis membrane.

14. The redox flow battery system of claim 11 where the membrane comprises functionalized polystyrene blended with polyvinyl chloride.

## Patentansprüche

1. Redox-Flow-Batterieelektrolyt, umfassend A) ein polares Lösungsmittel und B) ein DMTD-Metallsalzderivat der Formel (I): 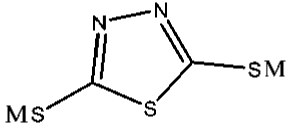 wobei M H oder ein Alkali- oder Erdalkalimetall wie Li, Na, K, Mg oder Ca ist, mit der Maßgabe, dass mindestens eines von M ein Alkali- oder Erdalkalimetall ist.

2. Redox-Flow-Batterieelektrolyt nach Anspruch 1, wobei beide M ein Erdalkalimetall sind.

3. Redox-Flow-Batterieelektrolyt nach Anspruch 1, wobei das polare Lösungsmittel Wasser umfasst.

4. Redox-Flow-Batterieelektrolyt nach einem der vorstehenden Ansprüche, wobei das polare Lösungsmittel Acetonitril umfasst.

5. Redox-Flow-Batterieelektrolyt nach einem der vorstehenden Ansprüche, wobei das polare Lösungsmittel ein organisches Lösungsmittel wie Carbonate, Ether, Ketone, Nitrile, Alkohole, Glykole, Amine, Amide, organische Lösungsmittel und Kombinationen davon umfasst.

6. Redox-Flow-Batterieelektrolyt nach einem der vorstehenden Ansprüche, wobei das DMTD-Metallsalzderivat davon von zu etwa 1 bis 50 Gew.-% der Elektrodenzusammensetzung vorhanden ist.

7. Redox-Flow-Batterieelektrolyt nach einem der vorstehenden Ansprüche, ferner umfassend eine unterstützende redoxaktive Spezies, die mindestens ein wasserlösliches monoalkyliertes DMTD-Derivat der Formel I enthält.

8. Redox-Flow-Batterieelektrolyt nach einem der vorstehenden Ansprüche, ferner umfassend eine unterstützende redoxaktive Spezies, umfassend mindestens eines von einem Lithiumsalz, Kaliumsalz, Natriumsalz, Ammoniumsalz und Kombinationen davon.

9. Redox-Flow-Batterieelektrolyt nach Anspruch 7 oder 8, wobei die unterstützende redoxaktive Spezies von zu etwa 0,1 bis etwa 20 Gew.-% der Elektrolytzusammensetzung vorhanden ist.

10. Redox-Flow-Batteriesystem, umfassend A) einen Katholyten, umfassend den Elektrolyten nach einem der Ansprüche 1 bis 9, und B) einen Anolyten, umfassend eine andere redoxaktive Spezies als das DMTD-Metallsalzderivat oder eine Mischung davon in einem polaren Lösungsmittel.

11. Redox-Flow-Batteriesystem nach Anspruch 10 ferner umfassend eine Ionenaustauschmembran oder mikroporöse Membran, die den Anolyten und den Katholyten trennt.

12. Redox-Flow-Batteriesystem nach Anspruch 11, wobei die Membran ein Fluorpolymer-Copolymer auf Basis von sulfoniertem Tetrafluorethylen ist.

13. Redox-Flow-Batteriesystem nach Anspruch 11, wobei die Membran eine Dialysemembran auf Zellulosebasis ist.

14. Redox-Flow-Batteriesystem nach Anspruch 11, wobei die Membran funktionalisiertes Polystyrol gemischt mit Polyvinylchlorid umfasst.

## Revendications

1. Électrolyte de batterie à flux redox comprenant A) un solvant polaire et B) un dérivé de sel métallique de DMTD de formule (I) : 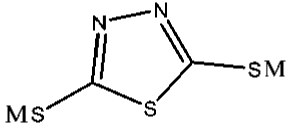 où M est H ou un métal alcalin ou alcalino-terreux, tel que Li, Na, K, Mg ou Ca, à condition qu'au moins un des M soit un métal alcalin ou alcalino-terreux.

2. Électrolyte de batterie à flux redox selon la revendication 1, dans lequel les deux M sont des métaux alcalino-terreux.

3. Électrolyte de batterie à flux redox selon la revendication 1, dans lequel le solvant polaire comprend de l'eau.

4. Électrolyte de batterie à flux redox selon l'une quelconque revendication précédente, dans lequel le solvant polaire comprend l'acétonitrile.

5. Électrolyte de batterie à flux redox selon l'une quelconque revendication précédente, dans lequel le solvant polaire comprend un solvant organique, tel que les carbonates, les éthers, les cétones, les nitriles, les alcools, les glycols, les amines, les amides, les solvants organiques et des combinaisons de ceux-ci.

6. Électrolyte de batterie à flux redox selon l'une quelconque revendication précédente, dans lequel le dérivé de sel métallique de DMTD est présent à hauteur d'environ 1 à 50 % en poids de la composition de l'électrolyte.

7. Électrolyte de batterie à flux redox selon l'une quelconque revendication précédente, comprenant en outre une espèce active redox de soutien contenant au moins un dérivé de DMTD mono-alkylé hydrosoluble de formule I.

8. Électrolyte de batterie à flux redox selon l'une quelconque revendication précédente, comprenant en outre une espèce active redox de soutien comprenant au moins l'un parmi sel de lithium, sel de potassium, sel de sodium, sel d'ammonium et des combinaisons de ceux-ci.

9. Électrolyte de batterie à flux redox selon la revendication 7 ou 8, dans lequel l'espèce active redox de soutien est présente à hauteur d'environ 0,1 à environ 20 % en poids de la composition de l'électrolyte.

10. Système de batterie à flux redox comprenant A) un catholyte comprenant l'électrolyte selon l'une quelconque des revendications 1 à 9, et B) un anolyte comprenant une espèce active redox autre que le dérivé de sel métallique de DMTD, ou un mélange de ceux-ci dans un solvant polaire.

11. Système de batterie à flux redox selon la revendication 10, comprenant en outre une membrane d'échange d'ions ou une membrane microporeuse séparant l'anolyte et le catholyte.

12. Système de batterie à flux redox selon la revendication 11, où la membrane est un copolymère-fluoropolymère sulfoné à base de tétrafluoroéthylène.

13. Système de batterie à flux redox selon la revendication 11, où la membrane est une membrane de dialyse à base de cellulose.

14. Système de batterie à flux redox selon la revendication 11, où la membrane comprend du polystyrène fonctionnalisé mélangé à du chlorure de polyvinyle.
